(19) Europäisches Patentamt
European Patent Office
Office européen des brevets

(11) Publication number : **0 585 042 A1**

# (12) EUROPEAN PATENT APPLICATION

(21) Application number : **93306513.8**

(22) Date of filing : **18.08.93**

(51) Int. Cl.$^5$ : **C12P 21/00,** A61K 37/02, // (C12P21/00, C12R1:38)

(30) Priority : **25.08.92 JP 225680/92**

(43) Date of publication of application : **02.03.94 Bulletin 94/09**

(84) Designated Contracting States : **AT BE CH DE DK ES FR GB GR IE IT LI LU MC NL PT SE**

(71) Applicant : **Shionogi & Co., Ltd.**
**1-8, Doshomachi 3-chome**
**Chuo-ku, Osaka 541 (JP)**

(72) Inventor : **Matsutani, Shigeru**
**3-24-3, Kimigaoka**
**Hashimoto-shi, Wakayama-ken (JP)**
Inventor : **Yoshida, Tadashi**
**3-4-4, Kofudai, Toyono-cho**
**Toyono-gun, Osaka-fu (JP)**
Inventor : **Sakazaki, Ryuji**
**726-163, Shimomaki, Kanmaki-cho**
**Kitakatsuragi-gun, Nara-ken (JP)**
Inventor : **Matsumoto, Koichi**
**1-6-1-401, Mukougaoka**
**Toyonaki-shi, Osaka-fu (JP)**

(74) Representative : **Allard, Susan Joyce et al**
**BOULT, WADE & TENNANT 27 Furnival Street**
**London EC4A 1PQ (GB)**

## (54) Antibiotic stalobacins.

(57) Novel antibiotic stalobacins A, B, C, D, E, F and G having physicochemical properties as shown in Tables 1, 2 and 3 are provided, which are excellent antibiotics showing marked effects on Gram-positive bacteria.

*Fig. 1*

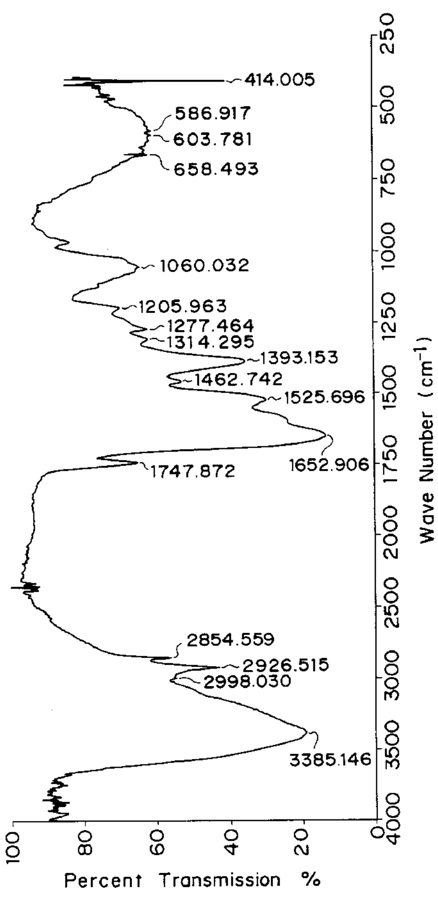


EP 0 585 042 A1

The present invention relates to novel antibiotics. In particular, this invention relates to antibiotic stalobacins A, B, C, D, E, F and G produced by Pseudomonas sp. PBJ-5,360.

It is well known that antibacterial activity of a particular antibiotic varies depending upon a nature of bacteria to be treated and that antibiotics may become ineffective to a certain class of bacteria because of the advent of resistant strains. The advent of multiple drug resistant bacteria has recently become a big problem. Accordingly, development of novel and effective antibiotics has been desired for solving this problem. Above all, many Gram-positive bacteria, such as staphylococcus, hemolytic streptococcus or the like, are resistant to antibiotics, and there is continuous need of the development of novel antibiotics having high potency to these Gram-positive bacteria.

It is disclosed in Japanese Patent Publication (Kokai) No. 303496/1990 that Pseudomonas sp. PBJ-5,360 produces a complex of several antibiotics which is therapeutically effective on Gram-positive bacteria. However, the publication does not disclose each of the components constituting said complex. The inventors of the present invention have succeeded in isolating each of the components and determining physico-chemical property of each component. The present invention is based on such findings.

Thus, the present invention provides antibiotic stalobacins selected from the group consisting of stalobacins A, B, C, D, E, F and G produced by Pseudomonas sp. PBJ-5,360. These antibiotics are peptide antibiotics which are produced by said Pseudomonas sp. PBJ-5,360. Stalobacins A - G (hereinafter sometimes referred to as merely "stalobacins") are produced in the form of a mixture of closely related analogs by cultivating said strain of Pseudomonas. Their antibacterial activities are much more potent than those of known antibiotics. Stalobacins A - G have physico-chemical properties as shown below in Tables 1 - 3.

Table 1

Physico-chemical Properties of Stalobacins A and B:

| | Stalobacin A | Stalobacin B |
|---|---|---|
| m.p. (°C)(as Na salt) | 240°C (dec.) | 240°C (dec.) |
| LSI-MS Maximal Peak (m/z) | 1483 | 1364 |
| HRLSI-MS | 1483.7347 | 1364.7165 |
| $(MH^+)$(m/z) | $C_{61}H_{107}N_{14}O_{28}$ | $C_{58}H_{102}N_{13}O_{24}$ |
| Theoretical Value | 1483.7372 | 1364.7154 |
| IR(KBr)($cm^{-1}$) | 3385,1748 | 3389,1748 |
| | 1653,1526 | 1653,1526 |
| UV($H_2O$) | Terminal Absorption | Terminal Absorption |
| (ε) at 210 nm | 36,300 | 35,500 |
| CD | $[\theta]_{196}$-77020 | $[\theta]_{193}$-70720 |
| | $[\theta]_{212}$+ 3159 | $[\theta]_{209}$+17630 |
| | $[\theta]_{231}$-33480 | $[\theta]_{231}$-29070 |
| | $[\theta]_{257}$+ 4028 | $[\theta]_{257}$+ 4905 |
| Retention Time (min.) in HPLC* | 7.8 | 8.4 |
| Amino Acid Analysis (molar ratio) | | |
| HyAsp[1)] | HyAsp(1) | HyAsp(1) |
| Asp | Asp(1) | Asp(1) |
| Ser | Ser(2) | Ser(1) |
| HyIle[2)] | HyIle(1) | HyIle(1) |
| Gly | Gly(1) | Gly(1) |
| Ala | Ala(1) | Ala(1) |
| Arg | - | Arg(1) |

| | | |
|---|---|---|
| | DNP - derivative | DNP - derivative |
| | m.p. >230°C (dec.) | m.p. >230°C (dec.) |
| | HRLSI-MS 1649.7378 | HRLSI-MS 1530.7169 |
| | $C_{67}H_{109}N_{16}O32$ | $C_{64}H_{103}N_{15}O_{28}$ |
| | Theoretical Value 1649.7386 | Theoretical Value 1530.7169 |

* Column : Develosil $5C_{18}$, 4.6ϕ x 250 mm;

Mobile Phase : $CH_3CN/2mM\ H_3PO_4$ (containing 50 mM-$Na_2SO_4$)

= 43/57;

Flow Rate : 1 ml/min.;

Chart Speed: 1 cm/min.

1) Hydroxyaspartic acid

2) Hydroxyisoleucine

Table 2

Physico-chemical Properties of Stalobacins C and D:

| | Stalobacin C | Stalobacin D |
|---|---|---|
| LSI-MS Maximal Peak (m/z) | 1396 | 1309 |
| HRLSI-MS | 1396.7061 | 1309.6706 |
| $(MH^+)$ (m/z) | $C_{58}H_{102}N_{13}O_{26}$ | $C_{55}H_{97}N_{12}O_{24}$ |
| Theoretical Value | 1396.7053 | 1309.6732 |
| IR(KBr)($cm^{-1}$) | 3411,1744 | 3418,1745 |
| | 1652,1528 | 1646,1525 |
| UV($H_2O$) | Terminal Absorption | Terminal Absorption |
| Retention Time (min.) in HPLC* | 8.8 | 10.0 |
| Amino Acid Analysis (molar ratio) | | |
| HyAsp[1)] | HyAsp(1) | HyAsp(1) |
| Asp | Asp(1) | Asp(1) |
| Ser | Ser(1) | - |
| HyIle[2)] | HyIle(1) | HyIle(1) |
| Gly | Gly(1) | Gly(1) |
| Ala | Ala(1) | Ala(1) |
| Arg | - | - |

* Column : Develosil $5C_{18}$, 4.6φ x 250 mm;

Mobile Phase : $CH_3CN$/2mM $H_3PO_4$ (containing 50 mM-$Na_2SO_4$) = 43/57;

Flow Rate : 1 ml/min.;

Chart Speed: 1 cm/min.

1) Hydroxyaspartic acid

2) Hydroxyisoleucine

Table 3

Physico-chemical Properties of Stalobacins E, F and G

| | Stalobacin E | Stalobacin F | Stalobacin G |
|---|---|---|---|
| m.p. (°C) (as Na salt) | 240°C dec. | 240°C dec. | 240°C dec. |
| LSI-MS Maximal Peak (m/z) | 1378 | 1497 | 1485 |
| HRLSI-MS | 1378.7318 | 1497.7532 | 1485.7529 |
| $(MH^+)$(m/z) | $C_{59}H_{104}N_{13}O_{24}$ | $C_{62}H_{109}N_{14}O_{28}$ | $C_{61}H_{109}N_{14}O_{28}$ |
| Theoretical Value | 1378.7311 | 1497.7529 | 1485.7529 |
| IR(KBr)($cm^{-1}$) | 3418,1748 | 3369,1746 | 3369,1746 |
| | 1651,1526 | 1653,1527 | 1654,1528 |
| (UV)($H_2O$) | Terminal Absorption | Terminal Absorption | Terminal Absorption |
| (∈) at 210 nm | 39,500 | 36,480 | 37,500 |
| CD | $[\theta]_{194}$-53530 | $[\theta]_{196}$-80050 | $[\theta]_{196}$-58600 |
| | $[\theta]_{208}$+15380 | $[\theta]_{212}$+ 1022 | $[\theta]_{212}$+10060 |
| | $[\theta]_{231}$-26750 | $[\theta]_{231}$-34490 | $[\theta]_{231}$-49830 |
| | $[\theta]_{256}$+4512 | $[\theta]_{257}$+4788 | $[\theta]_{257}$+ 5621 |
| Retention Time (min.)* in HPLC | 12.4 | 11.7 | 17.0 |

Amino Acid Analysis (Molar Ratio)

| | | | |
|---|---|---|---|
| HyAsp[1] | HyAsp(1) | HyAsp(1) | HyAsp(1) |
| Asp | Asp(1) | Asp(1) | Asp(1) |
| Ser | Ser(1) | Ser(2) | Ser(2) |
| HyIle[2] | HyIle(1) | HyIle(1) | HyIle(1) |
| Gly | Gly(1) | Gly(1) | Gly(1) |
| Ala | Ala(1) | Ala(1) | Ala(1) |
| Arg | Arg(1) | - | - |

* Column : Develosil $5C_{18}$, 4.6φ x 250 mm;

Mobile Phase : $CH_3CN$/2 mM $H_3PO_4$ (containing 50 mM-$Na_2SO_4$) = 43/57;

Flow Rate : 1 ml/min.;

Chart Speed: 1 cm/min.

1) Hydroxyaspartic acid

2) Hydroxyisoleucine

The antibiotic stalobacins of the present invention characterized by the above physical properties have been found to have excellent antibacterial activities in vitro and in vivo, showing potent effects especially on Gram-positive bacteria.

Thus, the stalobacins of the present invention show excellent antibacterial activities especially against Gram-positive bacteria, as shown in Table 6 below. The mechanism is considered to be based on the inhibition of cell wall synthesis.

No death was observed in acute toxicity test by intravenous administration of 300 mg/kg and 500 mg/kg to mice.

The antibiotic stalobacins of the present invention are produced by cultivating Pseudomonas sp. PBJ-5,360 belonging to Genus Pseudomonas aerobically in liquid medium containing assimilable carbon sources, nitrogen sources and mineral salts in a conventional manner. This bacterium has been identified as the above-mentioned strain by cultivating it according to the method as hereinafter described in Experiment 2 and examining comprehensively its morphology, culture properties, physiological and biochemical properties in reference to the description of Bergy's Manual of Systematic Bacteriology, Vol. 1 (1984). This strain may undergo a spontaneous or artificial mutation, and it is obvious to a person skilled in the art that such mutants should be included in the scope of the present invention, as far as they retain an ability to produce the stalobacins of the present invention. Thus, the present invention provides also Pseudomonas sp. PBJ-5,360 producing novel antibiotic stalobacins and mutants thereof having an ability to produce said antibiotic stalobacins. Pseudomonas sp. PBJ-5,360 was deposited under accession No. FERM P-10578 with National Institute of Bioscience and Human Technology, Higashi 1-1-3, Tsukubashi, Ibaraki Pref. Japan, on February 27, 1989, and then transferred to International Deposition under Budapest Treaty on June 17, 1993, and assigned accession No. FERM BP-4342.

Furthermore, the present invention provides a process for producing antibiotic stalobacins by cultivating such strain.

Ordinary compositions of medium and ordinary conditions used for conventional fermentation for producing antibiotics can be adopted. In principle, the medium includes carbon sources, nitrogen sources, mineral

sources and the like. If necessary, vitamines, precursors or the like can be added. Examples of carbon sources are glucose, starch, dextrin, glycerin, molasses, organic acids and the like, and these carbon sources may be used alone or in a mixture thereof. Examples of nitrogen sources are soybean powder, corn steep liquor, meat extract, yeast extract, cotton seed powder, peptone, wheat embryos, ammonium sulfate, ammonium nitrate and the like, and these nitrogen sources may be used alone or in a mixture thereof. Examples of mineral salts are calcium carbonate, sodium chloride, potassium chloride, magnesium sulfate, cupric sulfate, manganese chloride, zinc sulfate, cobalt chloride, various phosphates and the like. These materials may be added to a medium when required. A sufficient amount of antibiotic stalobacins is produced by cultivating Pseudomonas sp. PBJ-5,360 of the present invention in an appropriate medium at temperature from 20 to 35°C, preferably 25 - 29°C, for about 1 - 7 days. The antibiotic stalobacins are secreted into the culture medium. Accordingly, the product can easily be isolated and purified, if necessary, from the culture in a conventional manner. All of such procedures are well known to a person skilled in the art.

The antibiotic stalobacins of the present invention are believed to be useful for treating various infections, in particular, treating infections caused by multiple drug resistant Gram-positive bacteria, since they exhibit marked antibacterial activities in vitro and in vivo.

**Brief Description of the Drawings:**

Figure 1 shows IR spectrum of stalobacin A.
Figure 2 shows IR spectrum of stalobacin B.
Figure 3 shows IR spectrum of stalobacin C.
Figure 4 shows IR spectrum of stalobacin D.
Figure 5 shows IR spectrum of stalobacin E.
Figure 6 shows IR spectrum of stalobacin F.
Figure 7 shows IR spectrum of stalobacin G.
Figure 8 shows NMR spectrum of DNP-stalobacin A.
Figure 9 shows NMR spectrum of DNP-stalobacin B.
Figure 10 shows NMR spectrum of stalobacin E.
Figure 11 shows NMR spectrum of stalobacin F.
Figure 12 shows NMR spectrum of stalobacin G.
Figure 13 shows high performance liquid chromatogram demonstrating mutual conversion between stalobacins A and A' and stalobacins B and B'.

The present invention will be explained in more detail below by illustrating Examples and Experiments.

**Example 1.**

(a) Fermentation Step:

Eight hundreds ml of a medium (adjusted to pH 7 with 2 N-NaOH) consisting of 1.0% glucose, 0.5% yeast extract (Difco) and tap water in a 2 L Erlenmeyer flask was inoculated with Pseudomonas sp. PBJ-5,360 (kept at -80°C in 2 ml vial), and the resultant mixture was subjected to a shaking cultivation at 180 rpm with 70 mm of shaking breadth at temperature of 28°C for 20 hours. The culture (800 ml) was implanted to 25 L of a medium (adjusted to pH 7 with 2 N-NaOH) containing 1.0% glucose, 0.4% yeast extract (Difco), 1.0% molt extract (Difco), 0.1% polypeptone (Nippon Seiyaku) and tap water in 50 L jar fermenter, and the resultant mixture was cultivated with agitation at 200 rpm, with 20 L/min of aeration, under 0.35 $kg/cm^2$ of inner pressure, at temperature of 28°C for 20 hours.

Then, 13 L of this culture was implanted to 250 L of a medium (adjusted to pH 7 with 2 N-NaOH) consisting of 1.0% glucose, 1.0% molt extract (Difco), 1.0% powdery yeast, 0.2% tomato paste (Kagome), 1.0% β-cyclodextrin, 0.0008% defoaming agent P-2000 (Dainippon Ink) and tap water in a 500 L tank, and the resultant mixture was cultivated with 120 L/min of aeration, under 0.35 $kg/cm^2$ of inner pressure, with agitation at 320 rpm at a temperature of 28°C for 94 hours.

(b) Separation Step:

To 375 L of the culture obtained in the foregoing step was added 3.7 L of chloroform for sterilization. Then, the mixture was adjusted to pH 8.5 with 2 N-NaOH, mixed with 27 L of Diaion HP-20 (Mitsubishi Kasei Corporation) and stirred for 4 hours for adsorbing the active substances onto the resin. The mixture was allowed to stand for 1 hour, and the supernatant was discarded to recover Diaion HP-20. The resin was washed with

water and put in a glass column (inner diameter: 30 cm), and the active substances were eluted by a gradient of 40 L of 10 mM $Na_2HPO_4$ - 40 L of 90% methanol.

The fraction (21 L) showing antibacterial activity to S. aureus JC-1 was collected, adjusted to pH 7.0 with 2 N-HCl and concentrated in vacuo to 3 L. The concentrate was washed with 3 L of ethyl acetate to remove lipophilic materials. The ethyl acetate contained in the aqueous layer was evaporated in vacuo and the residue was charged onto MCI-GEL CHP 20P (Mitsubishi Kasei Corporation) column (5 cm I.D. x 51 cm) to adsorb the active substances, and the active substances were eluted stepwise with 2 mM $Na_2HPO_4$ - 80% MeOH·2 mM $Na_2HPO_4$. The eluted fractions were subjected to a HPLC analysis, and the fractions containing a group of stalobacins were collected, adjusted to pH 7.0 with 2 N-HCl, concentrated in vacuo and lyophilized. 3490 mg.

(c) Purification Step:

The First Purification Step:

Two lots of the crude extract obtained in the foregoing step were combined and dissolved in 230 ml of water (pH 8). The solution was adsorbed on Whatman DE-52 ($Cl^-$) ion exchange cellulose column (2 cm I.D. x 50 cm), and the column was washed with water. The column was subjected to a gradient elution with 600 ml of 10 mM $Na_2HPO_4$ (pH 7.5) - 600 ml of 1 M NaCl·10 mM $Na_2HPO_4$ (pH 7.5). The eluted fractions were subjected to a HPLC analysis, and 370 ml of the fractions containing a group of stalobacins were collected and adjusted to pH 7.0 with 2 N-HCl. The above fraction (370 ml) was adsorbed on MCI-GEL CHP 20P (Mitsubish Kasei Corporation) column (2 cm I.D. x 50 cm), which was then subjected to a gradient elution with 10 mM $Na_2HPO_4$ (pH 7.5) - 90% MeOH·10 mM $Na_2HPO_4$ (pH 7.5). The eluted fractions were subjected to a HPLC analysis, and the fractions containing a group of stalobacins were collected, adjusted to pH 7.0 with 2 N-HCl, concentrated in vacuo and lyophilized.

Table 4

| Fraction *No. | mg | Ingredient |
|---|---|---|
| 1 - 63 | Ca. 0 | |
| 63 - 75 | 1400 | Stalobacins B, E |
| 76 - 90 | 600 | Stalobacins A, B |
| 91 - 134 | 1250 | Stalobacins A, C, D, F, G |

*: 15 ml/Fraction

The Second Purification Step:

The fractions obtained above were purified by the preparative high performance liquid chromatography under the following conditions and then by the recycle preparative high performance liquid chromatography to give stalobacins A, B, C, D, E, F and G.

Fractions 63 - 75 (1400 mg) were dissolved in 40 ml of pure water, and the solution was subjected to the preparative high performance liquid chromatography with YMC ODS column (s-15/30μ, 5.0 x 5.0 cm, eluent: acetonitrile-2 mM phosphoric acid (50 mM $Na_2SO_4$) 47:53, flow rate: 100 ml/min, detection: 220 nm) to give a fraction (1.2 L) containing stalobacin B as a main ingredient and a fraction (2.0 L) containing stalobacin E as a main ingredient. Each fraction was neutralized with dilute aqueous sodium hydroxide and the acetonitrile was evaporated. The residue was adjusted to pH 7.4 - 7.5 and adsorbed on a column of MIC-GEL CHP-20P, which was washed with water and eluted with 60% aqueous acetone. Eluates were concentrated in vacuo and lyophilized to give 92 mg of stalobacin B fraction and 20 mg of stalobacin E fraction. The stalobacin B fraction (92 mg) obtained above was subjected to the recycle preparative high performance liquid chromatography with Develosil 5 $C_{18}$ column (2.0 x 25 cm, eluent: acetonitrile-2 mM phosphoric acid (50 mM $Na_2SO_4$) 47:53, flow

rate: 10 ml/min., detection: 220 nm)(twice repeated). The fraction separated was concentrated to remove the acetonitrile, and the residue was neutralized with dilute sodium hydroxide, adjusted to pH 7.3 - 7.5, adsorbed on a column of MIC-GEL CHP-20P, which was then washed with water and eluted with 60% aqueous acetone. The eluate was concentrated in vacuo to remove the acetone, and the residue was lyophilized to give 42 mg of pure stalobacin B. Further, the stalobacin E fraction (20 mg) obtained above was subjected to the recycle preparative high performance liquid chromatography (twice repeated) using Develosil 5 $C_{18}$ column in the same manner as described above to give 9 mg of pure stalobacin E.

Fractions 91 - 134 (1250 mg) obtained in the first purification step were treated in the same manner as above to give pure stalobacins A (83 mg), C (2 mg), D (3 mg), F (19 mg) and G (12 mg).

**Example 2.**

Preparation of DNP-stalobacins A and B: A mixture of stalobacins A and B (203 mg) was dissolved in 20 ml of 50% aqueous methanol, mixed with 800 mg of sodium bicarbonate and 4 ml of 10% ethanolic solution of 2,4-dinitrofluorobenzene and stirred at room temperature for 2 hours. The reaction mixture was adjusted to pH 3.0 with dilute hydrochloric acid, and the aqueous layer was washed with ether and ethyl acetate successively and extracted with n-butanol. The organic layer was dried over sodium sulfate and concentrated in vacuo. The residue (240 mg) was subjected to high performance liquid chromatography to give 65 mg of DNP-stalobacin A (reaction product of 2,4-dinitrofluorobenzene and stalobacin A)($t_R$ 18 min.) and 29 mg of DNP-stalobacin B ($t_R$ 20.2 min.).

Separation condition:

Column = Develosil ODS 2.0 x 25 cm
Eluent = 44% acetonitrile - 2mM phosphate buffer (pH 7.5, containing 50 mM $Na_2SO_4$)

| | |
|---|---|
| DNP-stalobacin A: | m.p. >230°C |
| | HR-LSIMS ($MH^+$) 1649.7378 |
| | $C_{67}H_{109}N_{16}O_{32}$ Theoretical Value 1649.7386 |
| DNP-stalobacin B: | m.p. >230°C |
| | HR-LSIMS ($MH^+$) 1530.7169 |
| | $C_{64}H_{104}N_{15}O_{28}$ Theoretical Value 1530.7169 |

Physico-chemical properties of stalobacins A, B, C, D, E, F, G, DNP-stalobacin A and DNP-stalobacin B obtained in Examples 1 - 2 were as shown in Tables 1, 2 and 3. IR spectra of stalobacins A - G were shown in Figs. 1 - 7, and NMR spectra of DNP-stalobacin A, DNP-stalobacin B, stalobacin E, stalobacin F and stalobacin G were shown in Figs. 8 - 12 of the accomanying drawings, respectively. Putative partial structures of DNP-stalobacin A and DNP-stalobacin B were shown below.

DNP – Stalobasin A

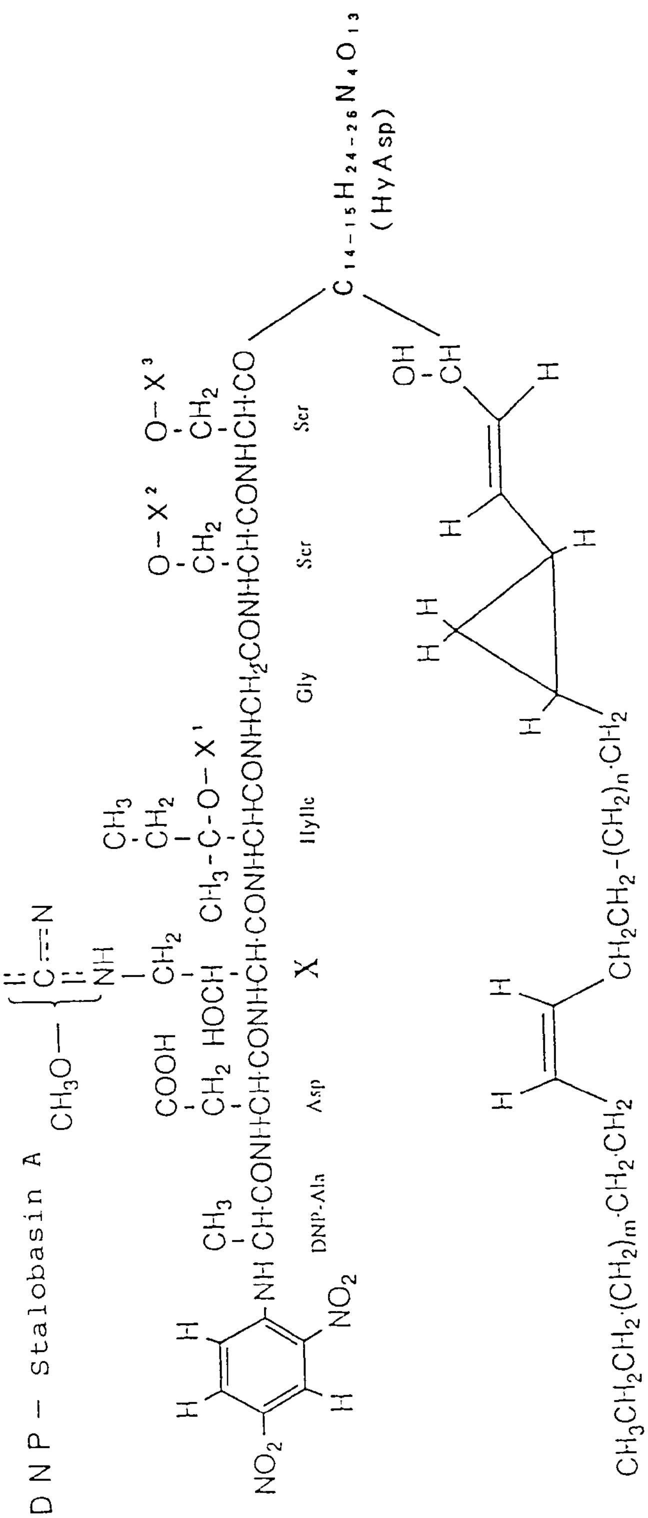

$m+n=3 \sim 4$

DNP - Stalobasin B

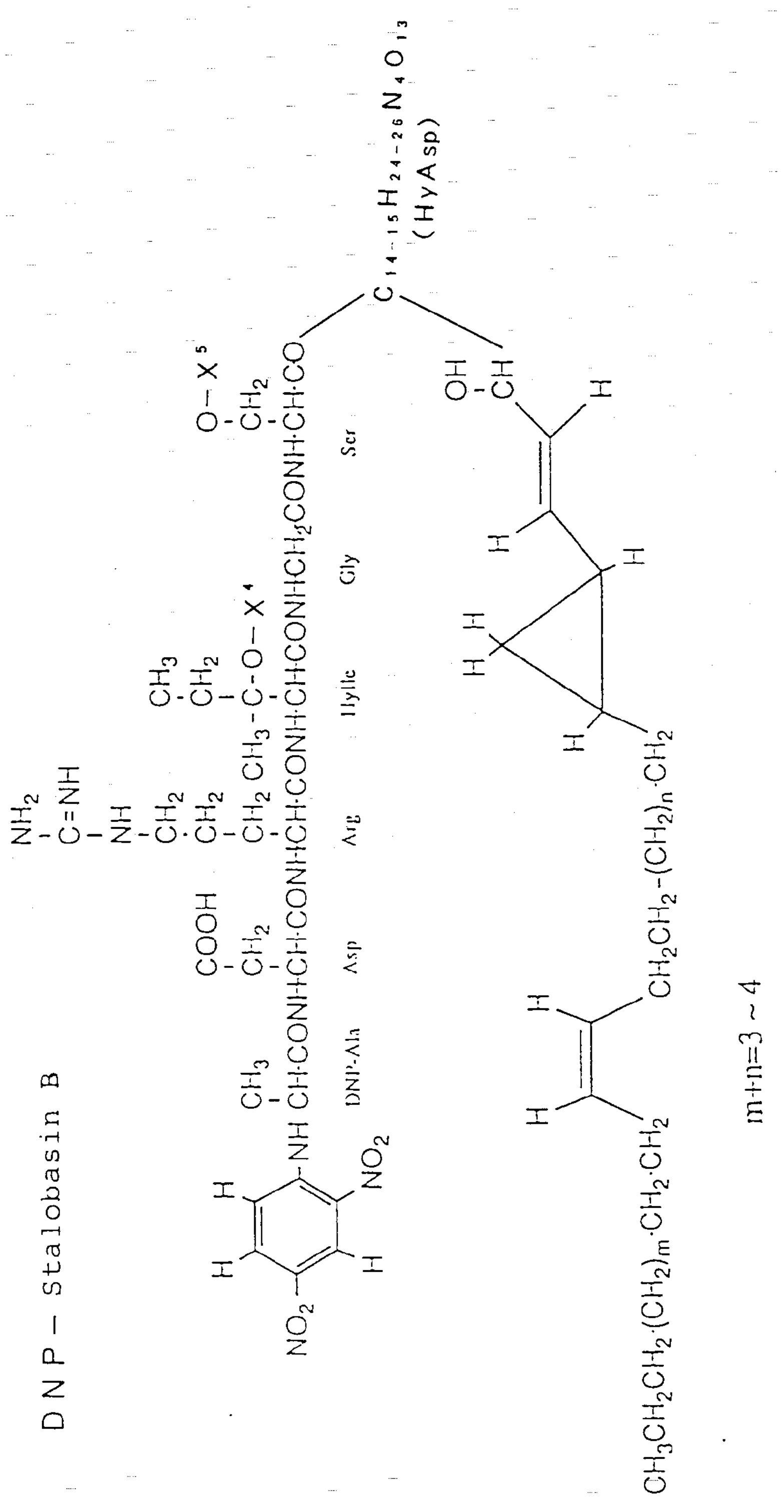

m+n=3 ~ 4

In the chemical structures above, HyAsp and Hylle represent hydroxyaspartic acid and hydroxyisoleucine, respectively, and (HyAsp) represents a moiety including hydroxyaspartic acid, and $X^1$, $X^2$, $X^3$, $X^4$ and $X^5$ represent unidentified moieties. There is possibility that all or some of $X^1$, $X^2$, $X^3$, $X^4$ and $X^5$ represent a single bond and they connect to the $C_{14-15}H_{24-25}N_4O_{13}$ (HyAsp) moiety in the molecule.

<u>Unique Properties of Stalobacins A and B:</u>

(1) As shown in Figure 13 (a), a mixture of stalobacins A and B was subjected to high performance liquid

chromatography, whereby total 4 peaks corresponding to A, its isomer A', B and its isomer B' were observed. However, when this mixture was allowed to stand for 24 hours and then subjected again to high performance liquid chromatography, only two peaks corresponding to A and B were observed with disappearance of peaks of A' and B'. Each of A and B was isolated and again subjected to liquid chromatography, whereby two peaks corresponding to A and A' were observed for A and two peaks corresponding B and B' were observed for B (Fig. 13(b)). Each of these fractions was allowed to stand for one day, whereby the peaks of A' and B' disappeared again (Fig. 13(c)).

(2) Stalobacin B has a molecular formula $C_{58}H_{101}N_{13}O_{24}$ and an IR absorption at 1747 $cm^{-1}$. It is treated with dilute NaOH to give a compound of molecular formula $C_{58}H_{103}N_{13}O_{25}$. The antibacterial activity markedly lowered with the disappearance of the absorption at 1747 $cm^{-1}$.

(3) Table 5 shows the aspect of the mutual conversion between A and A', when stalobacin A was dissolved in aqueous solution of various pH.

Table 5

| Time after dissolution | Composition (%)* | | | | | |
|---|---|---|---|---|---|---|
| | pH3.0 | | pH7.0 | | pH8.0 | |
| | A | A' | A | A' | A | A' |
| 0 | 82.2 | 17.8 | 81.9 | 18.1 | 82.1 | 17.9 |
| 1 hour | 96.0 | 3.1 | 87.9 | 12.1 | 87.3 | 12.7 |
| 2 hour | 100 | 0 | 100 | 0 | 100 | 0 |
| 3 hour | 100 | 0 | 100 | 0 | 100 | 0 |

*: High Performance Liquid Chromatography

**Experiment 1** Antibacterial Activity in vitro and in vivo:

1) In vitro Antibacterial Activity:

Antibacterial activity in vitro of antibiotic stalobacins A - G obtained in Example 1 was assayed by the agar dilution method. Table 6 shows the results.

Table 6

| | Stalobacin (μg/ml, $10^6$cfu/ml) | | | | | | |
|---|---|---|---|---|---|---|---|
| | A | B | C | D | E | F | G |
| Gram-positive bacteria | | | | | | | |
| S. aureus FDA JC-1 | 0.025 | 0.1 | 0.08 | 0.01 | 0.025 | 0.02 | 0.1 |
| S. faecalis SR1004 | 0.1 | 0.39 | 0.31 | 0.08 | 0.39 | 0.1 | 0.2 |
| S. aureus 3626 (MRSA) | 0.05 | 0.1 | 0.16 | 0.04 | 0.025 | 0.05 | 0.2 |

2) In vivo Antibacterial Activity:

Antibacterial activity in vivo of stalobacin A was assayed. Mice were intraperitoneally challenged with infectious bacteria. One hour after the challenge, the test compound was subcutaneously administered. $ED_{50}$ value was calculated on the basis of survival ratio on 7th day after the challenge. MIC was determined according to the agar dilution method. Table 7 shows the results.

Table 7

| Protective Effect of Stalobacin A in Mice Systemically Infected: | | |
|---|---|---|
| | $ED_{50}$(mg/kg) | MIC (μg/ml) |
| | Stalobacin A | Stalobacin A |
| S. aureus Smith | 0.17 | 0.006 |
| S. aureus SR3637 (H-MRSA) | 0.27 | 0.025 |
| S. pyogenes C-203 | 0.12 | 0.013 |
| S. pneumoniae Type I | 0.058 | 0.006 |

**Experiment 2** Bacteriological Properties of PBJ-5,360:

Bacteriological properties of PBJ-5,360 of the present invention are shown below. PBJ-5,360 was isolated from the soil collected in Kyoto, Japan.

Cultivation was effected at 28°C in principle.

A. Morphology:

It is a Gram-negative rod. Its size is 0.3 - 0.5 (μ) x 0.8 - 1.3 (μ). It vigorously moves with one or more polar flagella.

B. Characteristics of Culture

1) Cultivation in meat infusion medium:

Growth of the bacteria was hardly observed. Off white translucent precipitates formed very slightly at the bottom of the test tube.

2) Meat infusion agar stab culture:

Growth in thread form or small nipple form along the stab line was observed. Neither evolution of gas nor production of pigment was observed. Reddish thin bacterial plaque appeared on the surface, but this bacterial plaque became translucent and light brown with the lapse of time and verrucose projections were observed in several places. It is an aerobic bacterium.

3) Meat infusion agar slant culture:

The growth of the bacteria was not so rapid and began at 28°C after two days (observed with naked eyes). The bacteria grew in thread form, and the bacterial plaque was translucent and light yellow with flat swelling having a spotty appearance. The periphery was whole peripheral. Then, the bacterial plaque grew favorably in thread form or verrucose form with gloss. Thus, a wet slightly reddish translucent brown bacterial plaque was obtained. The periphery was wavy or long wavy. Production of any gas or pigment was not observed.

4) Meat infusion gelatin stab culture.

Cultivation was effected at room temperature (22 - 25°C). The gelatin was liquefied.

5) Cultivation on the meat infusion agar plane medium:

The growth of the bacteria was not so rapid. The colony became visible at 28°C after two days. The colony was initially small, spotty, translucent and brown with a whole periphery. The colony was too small to be observed about its swelling. Then, the colony grew in a spotty or circular form and with whole periphery and the swelling was flat or convex circular. The colony was translucent and brown with gloss. Neither gas nor soluble pigment was produced.

6) Characteristics in litmus milk culture:

An acid formation and peptonization occurred, but the reactions occurred rather slowly. Thus, the reaction began after 14 days. No gas evolved. Sometimes grey reddish purple thin pellicle formed on the surface. The upper layer was translucent and reddish purple, and the lower layer was opaque and purplish beige. The precipitate was reddish purple close to beige.

C. Physiological and Biochemical Properties:

1) Catalase test: positive
2) Oxidase test: positive
3) OF-test: negative (showing to be alkaline)
4) Hemolytic test: positive (weakly)

5) Viability at 5°C: negative
6) Production of $H_2S$: negative
7) Reducing ability for nirate: positive
8) Denitrification: negative (although no nitrogen gas was evolved, it seemed to reduce $NO_2^-$.)
9) Availability of citric acid: negative (Christensen medium and Simons medium)
10) Growth on NAC agar medium: negative (nonviable)
11) Production of indole: negative
12) Voges-Proskauer reaction (Voges-Proskauer test): negative
13) Methylred test: negative
14) Hydrolyzing ability for arginine: positive
15) Decarboxylation ability for lysine: positive
16) Decarboxylation ability for ornithine: positive
17) Hydrolyzing ability for esculine: negative
18) DNase test: negative
19) Hydrolyzing ability for starch: negative
20) ONPG test (cultivated at 37°C): negative
21) Acylamidase test: negative
22) Phosphatase test: positive
23) Hydrolyzing ability for chitin: negative
24) Productivity of levan from sucrose: positive
25) Productivity of acids and gas from sugars:
Neither acids nor gases were produced from the following 13 sugars: glucose, fructose, galactose, mannose, xylose, arabinose, maltose, lactose, ramnose, sucrose, cellobiose, trehalose, mannit.
26) Accumulation of poly-β-hydroxybutyrate in the cell: negative
27) Availability of carbon sources: On the medium containing minerals, glucose and calcium 2-keto-gluconate can be used as a sole carbon source for the formation of the cells. In this case, it seemed that specific vitamines for growth were not required. On the other hand, D-(+)-trehalose, DL-arginine, geraniol, β-alanine, L-valine and innosit can not be used.
28) G + C mole % (HPLC method): 60.4% (A + T mole % = 39.6%)

In view of the properties above, the present bacterium is an aerobic Gram-negative rod and moves actively in a liquid medium using one or more polar flagella. It is positive for catalase and contains oxidase. It was negative for OF-test (showing to be alkaline). In view of these observations, it is apparent that the present bacterium belongs to Genus Pseudomanas in Family Pseudomaona.

When the inventors compared the above properties with those of the bacterial complexes which are incapable of accumulating poly-β-hydroxybutyrate (PHB) in their cells, which complexes are described in Bergey's Manual of Systematic Bacteriology, Vol. 1 (1984) on Genus Pseudomonas, the present inventors failed to find any bacterium having those properties consistent or analogous to the properties described above. Therefore, this bacterium may be a new strain of Pseudomonas, and it appears a considerably unusual strain of Pseudomonas because it hydrolyzes arginine and decarboxylates lysine and ornithine. The G + C mole % value of 60.4% indicates that the strain belongs to a group having lower G + C value in Pseudomonas. Thus, in view of the various properties above, the present bacterium has been designated as Pseudomonas sp. PBJ-5,360.

## Claims

**1.** A Stalobacin selected from stalobacin A, B, C, D, E, F or G having physico-chemical properties as shown below in Tables 1, 2 or 3:

Table 1

Physico-chemical Properties of Stalobacins A and B:

| | Stalobacin A | Stalobacin B |
|---|---|---|
| m.p. (°C)(as Na salt) | 240°C (dec.) | 240°C (dec.) |
| LSI-MS Maximal Peak (m/z) | 1483 | 1364 |
| HRLSI-MS | 1483.7347 | 1364.7165 |
| ($MH^+$)(m/z) | $C_{61}H_{107}N_{14}O_{28}$ | $C_{58}H_{102}N_{13}O_{24}$ |
| Theoretical Value | 1483.7372 | 1364.7154 |
| IR(KBr)($cm^{-1}$) | 3385,1748 | 3389,1748 |
| | 1653,1526 | 1653,1526 |
| UV($H_2O$) | Terminal Absorption | Terminal Absorption |
| (ε) at 210 nm | 36,300 | 35,500 |
| CD | $[\theta]_{196}$-77020 | $[\theta]_{193}$-70720 |
| | $[\theta]_{212}$+ 3159 | $[\theta]_{209}$+17630 |
| | $[\theta]_{231}$-33480 | $[\theta]_{231}$-29070 |
| | $[\theta]_{257}$+ 4028 | $[\theta]_{257}$+ 4905 |
| Retention Time (min.) in HPLC* | 7.8 | 8.4 |
| Amino Acid Analysis (molar ratio) | | |
| HyAsp[1)] | HyAsp(1) | HyAsp(1) |
| Asp | Asp(1) | Asp(1) |
| Ser | Ser(2) | Ser(1) |

| | | |
|---|---|---|
| HyIle[2)] | HyIle(1) | HyIle(1) |
| Gly | Gly(1) | Gly(1) |
| Ala | Ala(1) | Ala(1) |
| Arg | - | Arg(1) |
| | DNP - derivative | DNP - derivative |
| | m.p. >230°C (dec.) | m.p. >230°C (dec.) |
| | HRLSI-MS 1649.7378 | HRLSI-MS 1530.7169 |
| | $C_{67}H_{109}N_{16}O32$ | $C_{64}H_{103}N_{15}O_{28}$ |
| | Theoretical Value 1649.7386 | Theoretical Value 1530.7169 |

* Column : Develosil $5C_{18}$, 4.6ϕ x 250 mm;

Mobile Phase : $CH_3CN$/2mM $H_3PO_4$ (containing 50 mM-$Na_2SO_4$) = 43/57;

Flow Rate : 1 ml/min.;

Chart Speed: 1 cm/min.

1) Hydroxyaspartic acid

2) Hydroxyisoleucine

Table 2

Physico-chemical Properties of Stalobacins C and D:

| | Stalobacin C | Stalobacin D |
|---|---|---|
| LSI-MS Maximal Peak (m/z) | 1396 | 1309 |
| HRLSI-MS | 1396.7061 | 1309.6706 |
| ($MH^+$)(m/z) | $C_{58}H_{102}N_{13}O_{26}$ | $C_{55}H_{97}N_{12}O_{24}$ |
| Theoretical Value | 1396.7053 | 1309.6732 |
| IR(KBr)($cm^{-1}$) | 3411,1744 | 3418,1745 |
| | 1652,1528 | 1646,1525 |
| UV($H_2O$) | Terminal Absorption | Terminal Absorption |
| Retention Time (min.) in HPLC* | 8.8 | 10.0 |
| Amino Acid Analysis (molar ratio) | | |
| HyAsp[1)] | HyAsp(1) | HyAsp(1) |
| Asp | Asp(1) | Asp(1) |
| Ser | Ser(1) | - |
| HyIle[2)] | HyIle(1) | HyIle(1) |
| Gly | Gly(1) | Gly(1) |
| Ala | Ala(1) | Ala(1) |
| Arg | - | - |

* Column : Develosil $5C_{18}$, 4.6φ x 250 mm;

Mobile Phase : $CH_3CN$/2mM $H_3PO_4$ (containing 50 mM-$Na_2SO_4$) = 43/57;

Flow Rate : 1 ml/min.;

Chart Speed: 1 cm/min.

1) Hydroxyaspartic acid

2) Hydroxyisoleucine

Table 3

Physico-chemical Properties of Stalobacins E, F and G

| | Stalobacin E | Stalobacin F | Stalobacin G |
|---|---|---|---|
| m.p. (°C) (as Na salt) | 240°C dec. | 240°C dec. | 240°C dec. |
| LSI-MS Maximal Peak (m/z) | 1378 | 1497 | 1485 |
| HRLSI-MS | 1378.7318 | 1497.7532 | 1485.7529 |
| $(MH^+)$(m/z) | $C_{59}H_{104}N_{13}O_{24}$ | $C_{62}H_{109}N_{14}O_{28}$ | $C_{61}H_{109}N_{14}O_{28}$ |
| Theoretical Value | 1378.7311 | 1497.7529 | 1485.7529 |
| IR(KBr)($cm^{-1}$) | 3418,1748 | 3369,1746 | 3369,1746 |
| | 1651,1526 | 1653,1527 | 1654,1528 |
| (UV)($H_2O$) | Terminal Absorption | Terminal Absorption | Terminal Absorption |
| (∈) at 210 nm | 39,500 | 36,480 | 37,500 |
| CD | $[\theta]_{194}$-53530 | $[\theta]_{196}$-80050 | $[\theta]_{196}$-58600 |
| | $[\theta]_{208}$+15380 | $[\theta]_{212}$+ 1022 | $[\theta]_{212}$+10060 |
| | $[\theta]_{231}$-26750 | $[\theta]_{231}$-34490 | $[\theta]_{231}$-49830 |
| | $[\theta]_{256}$+4512 | $[\theta]_{257}$+4788 | $[\theta]_{257}$+ 5621 |
| Retention Time (min.)* in HPLC | 12.4 | 11.7 | 17.0 |
| Amino Acid Analysis (Molar Ratio) | | | |
| HyAsp[1)] | HyAsp(1) | HyAsp(1) | HyAsp(1) |

| | | | |
|---|---|---|---|
| Asp | Asp(1) | Asp(1) | Asp(1) |
| Ser | Ser(1) | Ser(2) | Ser(2) |
| HyIle[2)] | HyIle(1) | HyIle(1) | HyIle(1) |
| Gly | Gly(1) | Gly(1) | Gly(1) |
| Ala | Ala(1) | Ala(1) | Ala(1) |
| Arg | Arg(1) | - | - |

* Column : Develosil $5C_{18}$, 4.6ϕ x 250 mm;

Mobile Phase : $CH_3CN$/2 mM $H_3PO_4$ (containing 50 mM-$Na_2SO_4$) = 43/57;

Flow Rate : 1 ml/min.;

Chart Speed: 1 cm/min.

1) Hydroxyaspartic acid

2) Hydroxyisoleucine

2. Stalobacin A, B, C, D, E, F, or G as claimed in claim 1 which is in substantially pure form.

3. A mixture of two or more of the stalobacins as claimed in claim 2.

4. Stalobacins as claimed in any one of claims 1 to 3 produced by Pseudomonas sp PBJ-5,360 or mutants thereof.

5. A process for the production of antibiotic stalobacins as claimed in any one of claims 1 to 3 comprising cultivating cells of Pseudomonas sp PBJ-5,360 and/or mutant cells thereof and separating and recovering the antibiotic stalobacins from the culture.

6. A pharmaceutical composition comprising stalobacins as claimed in any one of claims 1 to 3 together with a pharmaceutically acceptable carrier or diluent

7. Stalobacins as claimed in any one of claims 1 to 3 for use as an active pharmaceutical substance.

8. Stalobacins as claimed in any one of claims 1 to 3 for use in treatment of infections or diseases caused by gram positive bacteria.

Fig. 1

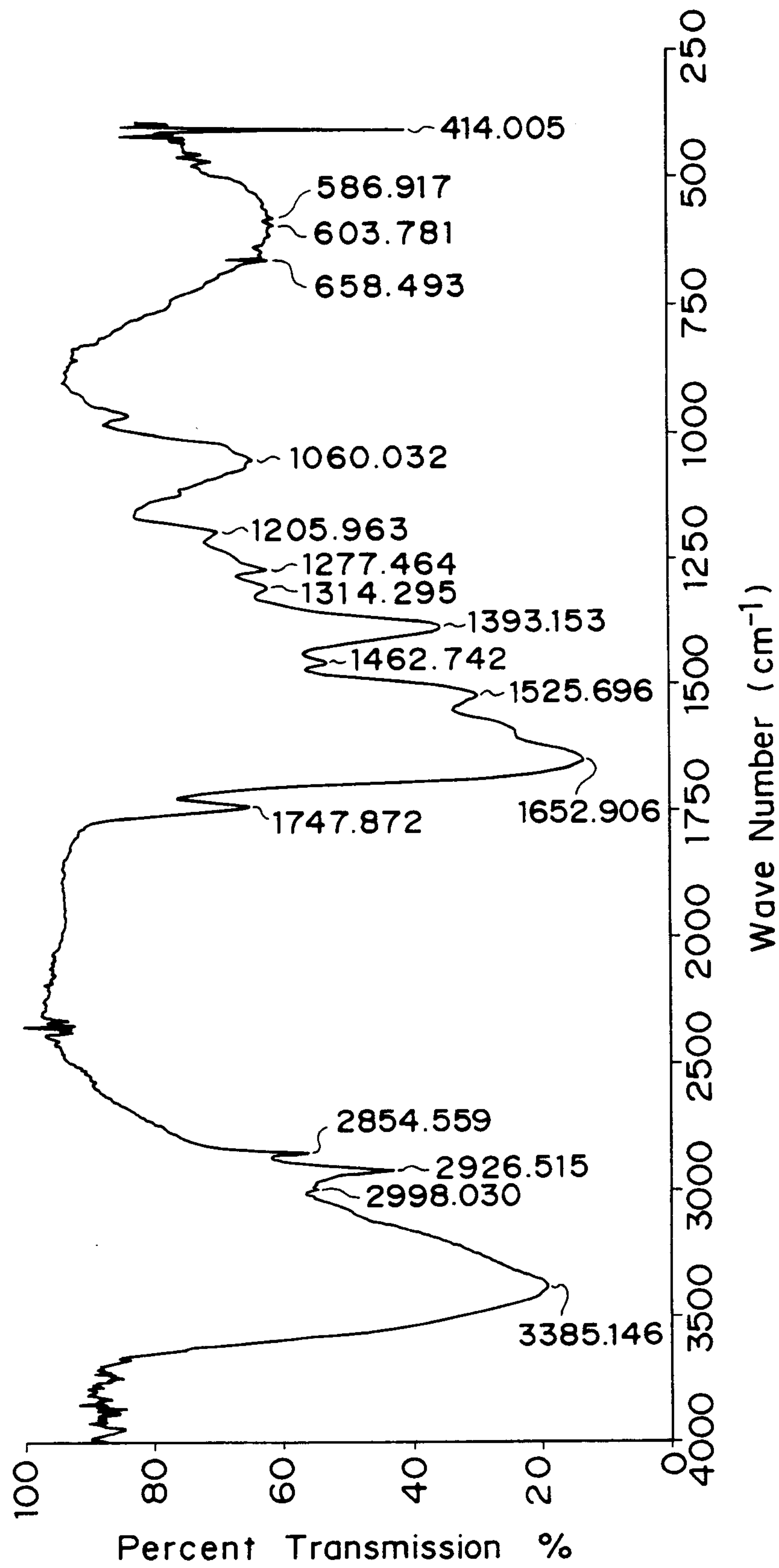
414.005
586.917
603.781
658.493
1060.032
1205.963
1277.464
1314.295
1393.153
1462.742
1525.696
1652.906
1747.872
2854.559
2926.515
2998.030
3385.146
Wave Number (cm$^{-1}$)
Percent Transmission %
100
80
60
40
20
0
4000
3500
3000
2500
2000
1750
1500
1250
1000
750
500
250

Fig. 2

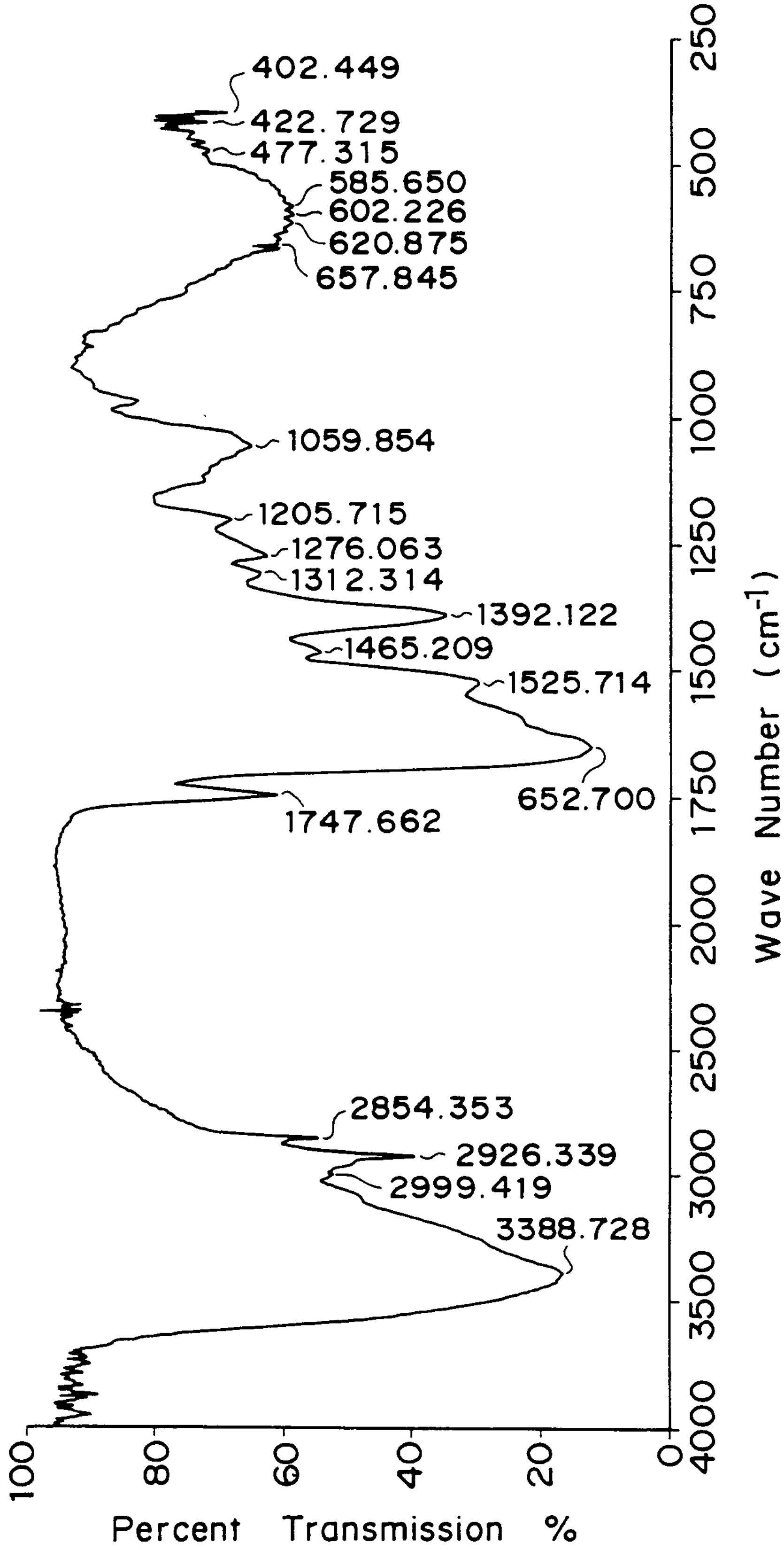
402.449
422.729
477.315
585.650
602.226
620.875
657.845
1059.854
1205.715
1276.063
1312.314
1392.122
1465.209
1525.714
652.700
1747.662
2854.353
2926.339
2999.419
3388.728
Wave Number (cm$^{-1}$)
4000
3500
3000
2500
2000
1750
1500
1250
1000
750
500
250
Percent Transmission %
100
80
60
40
20
0

Fig. 3

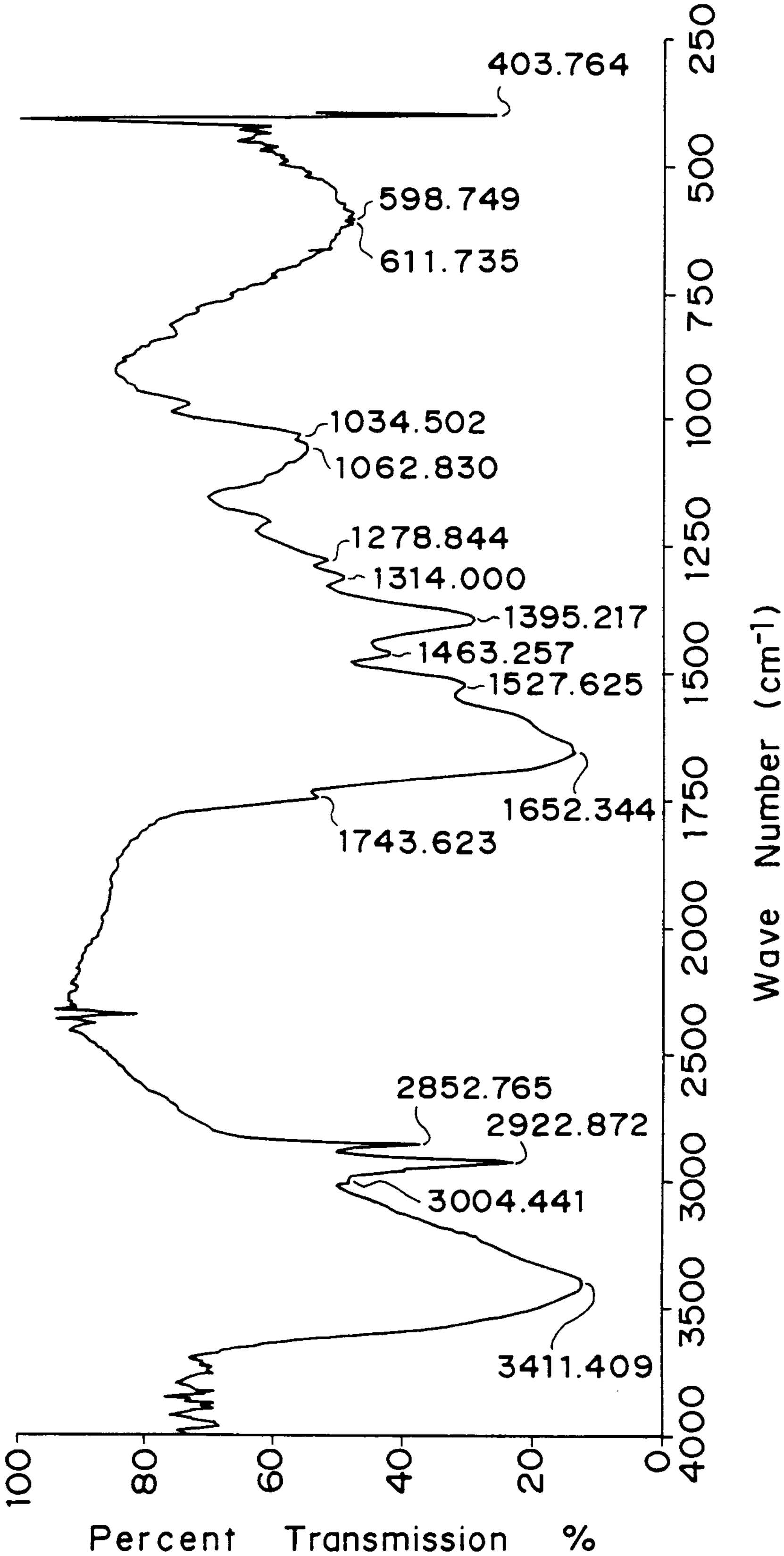
403.764
598.749
611.735
1034.502
1062.830
1278.844
1314.000
1395.217
1463.257
1527.625
1652.344
1743.623
2852.765
2922.872
3004.441
3411.409
250
500
750
1000
1250
1500
1750
2000
2500
3000
3500
4000
Wave Number (cm$^{-1}$)
100
80
60
40
20
0
Percent Transmission %

Fig. 4

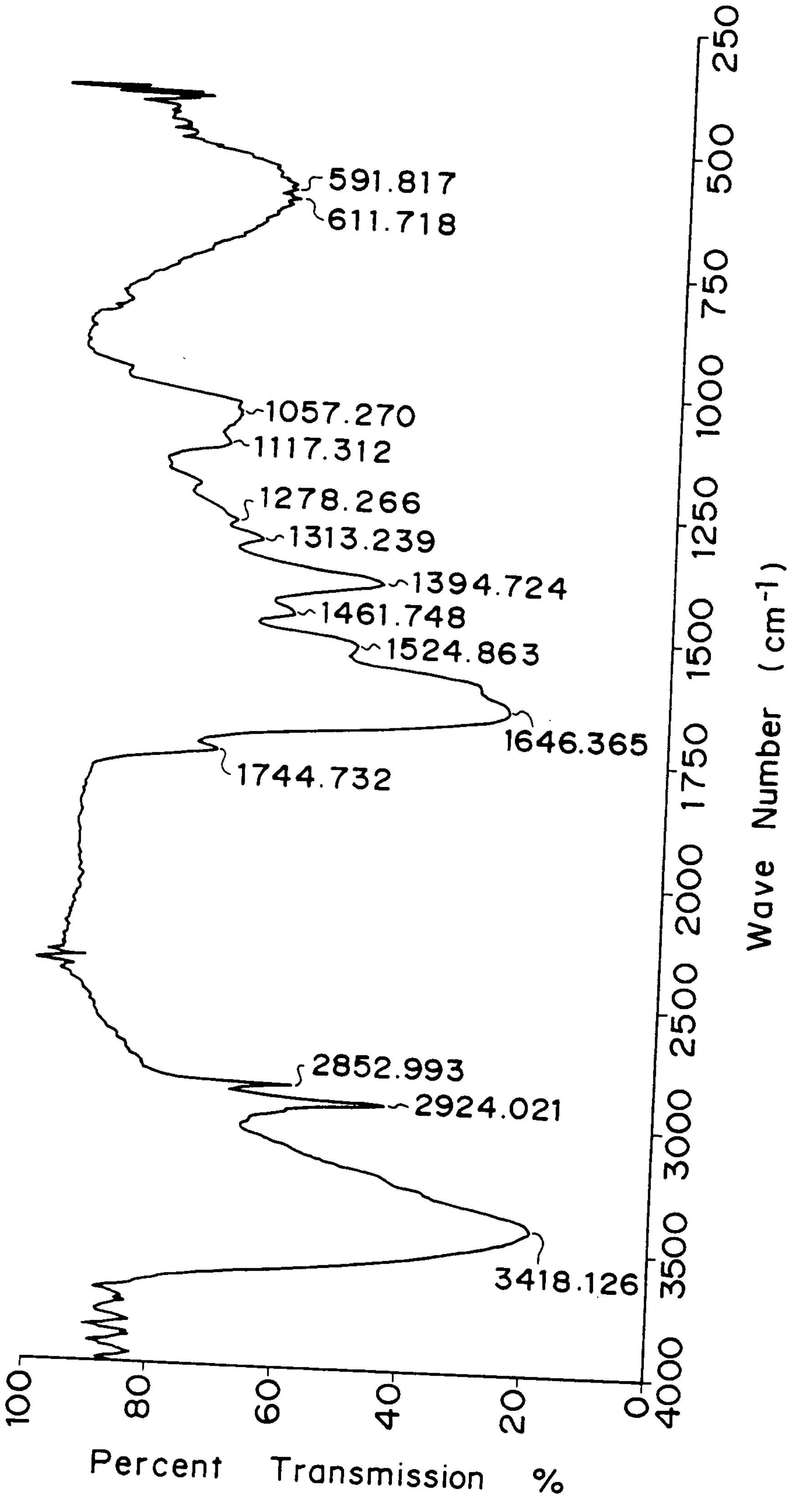
591.817
611.718
1057.270
1117.312
1278.266
1313.239
1394.724
1461.748
1524.863
1646.365
1744.732
2852.993
2924.021
3418.126
Wave Number (cm-1)
4000
3500
3000
2500
2000
1750
1500
1250
1000
750
500
250
Percent Transmission %
100
80
60
40
20
0

*F i g . 5*

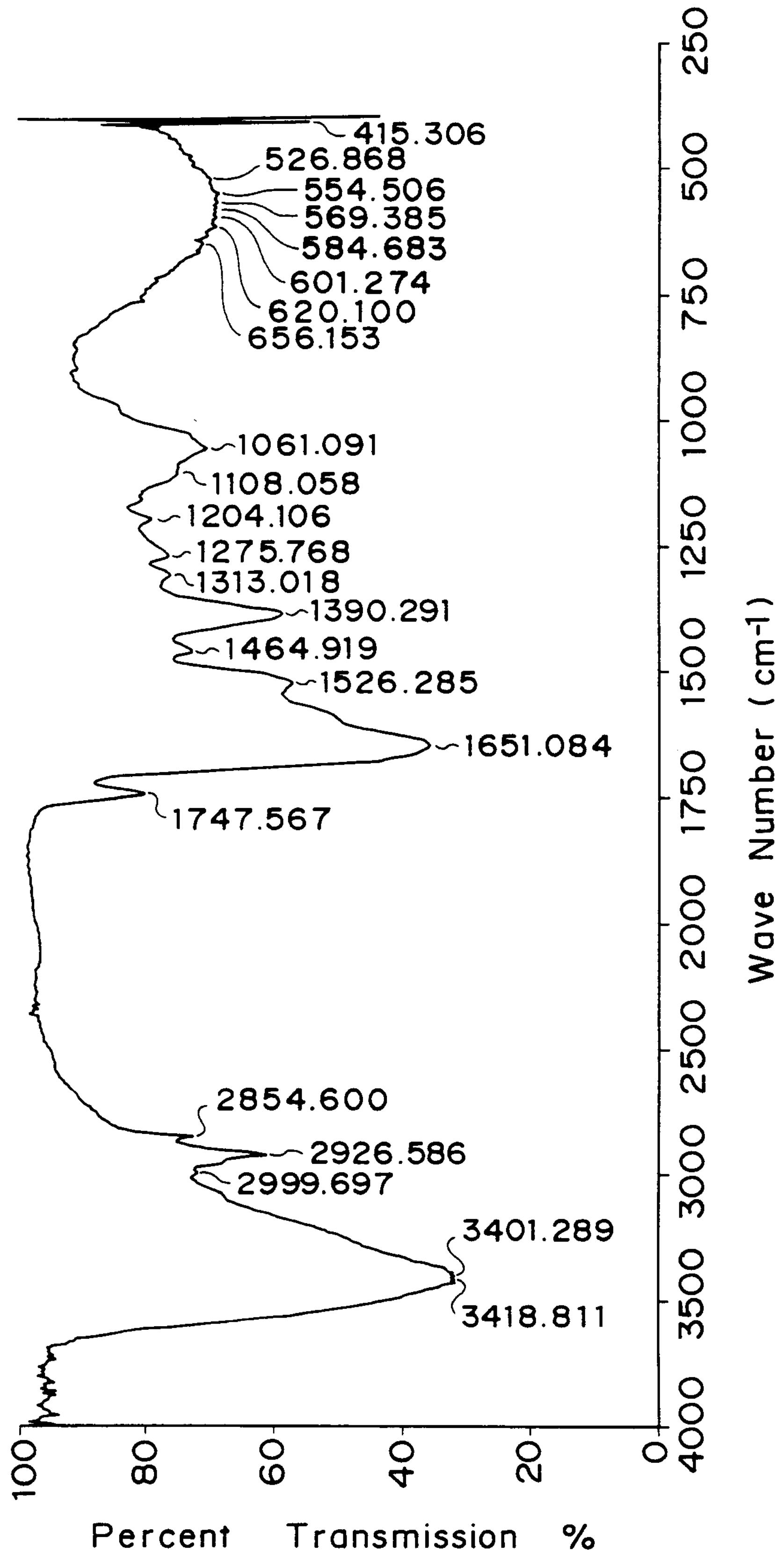
415.306
526.868
554.506
569.385
584.683
601.274
620.100
656.153
1061.091
1108.058
1204.106
1275.768
1313.018
1390.291
1464.919
1526.285
1651.084
1747.567
2854.600
2926.586
2999.697
3401.289
3418.811
250
500
750
1000
1250
1500
1750
2000
2500
3000
3500
4000
Wave Number ( cm-1)
100
80
60
40
20
0
Percent Transmission %

Fig. 6

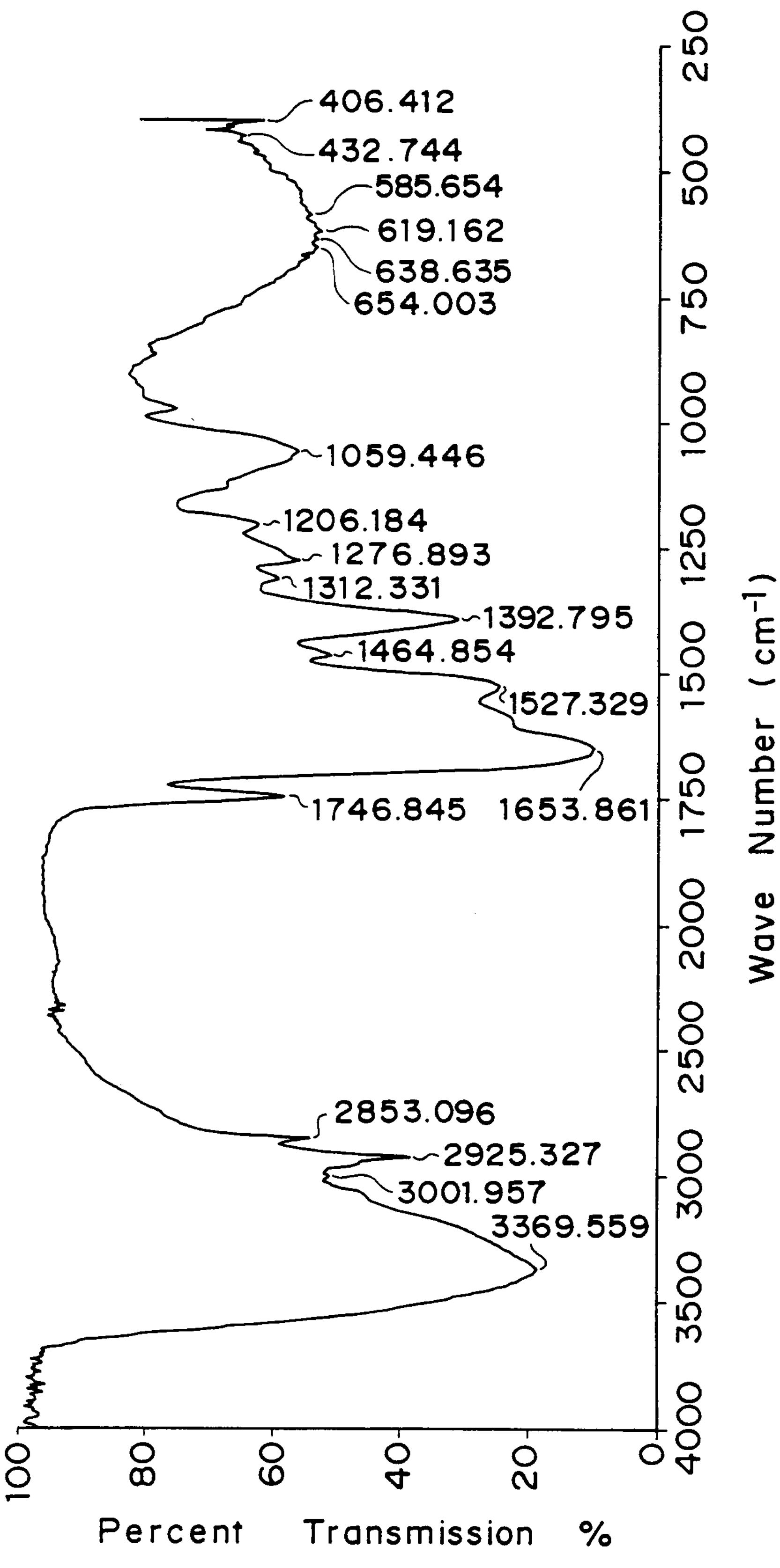
406.412
432.744
585.654
619.162
638.635
654.003
1059.446
1206.184
1276.893
1312.331
1392.795
1464.854
1527.329
1653.861
1746.845
2853.096
2925.327
3001.957
3369.559
250
500
750
1000
1250
1500
1750
2000
2500
3000
3500
4000
Wave Number (cm-1)
100
80
60
40
20
0
Percent Transmission %

Fig.7

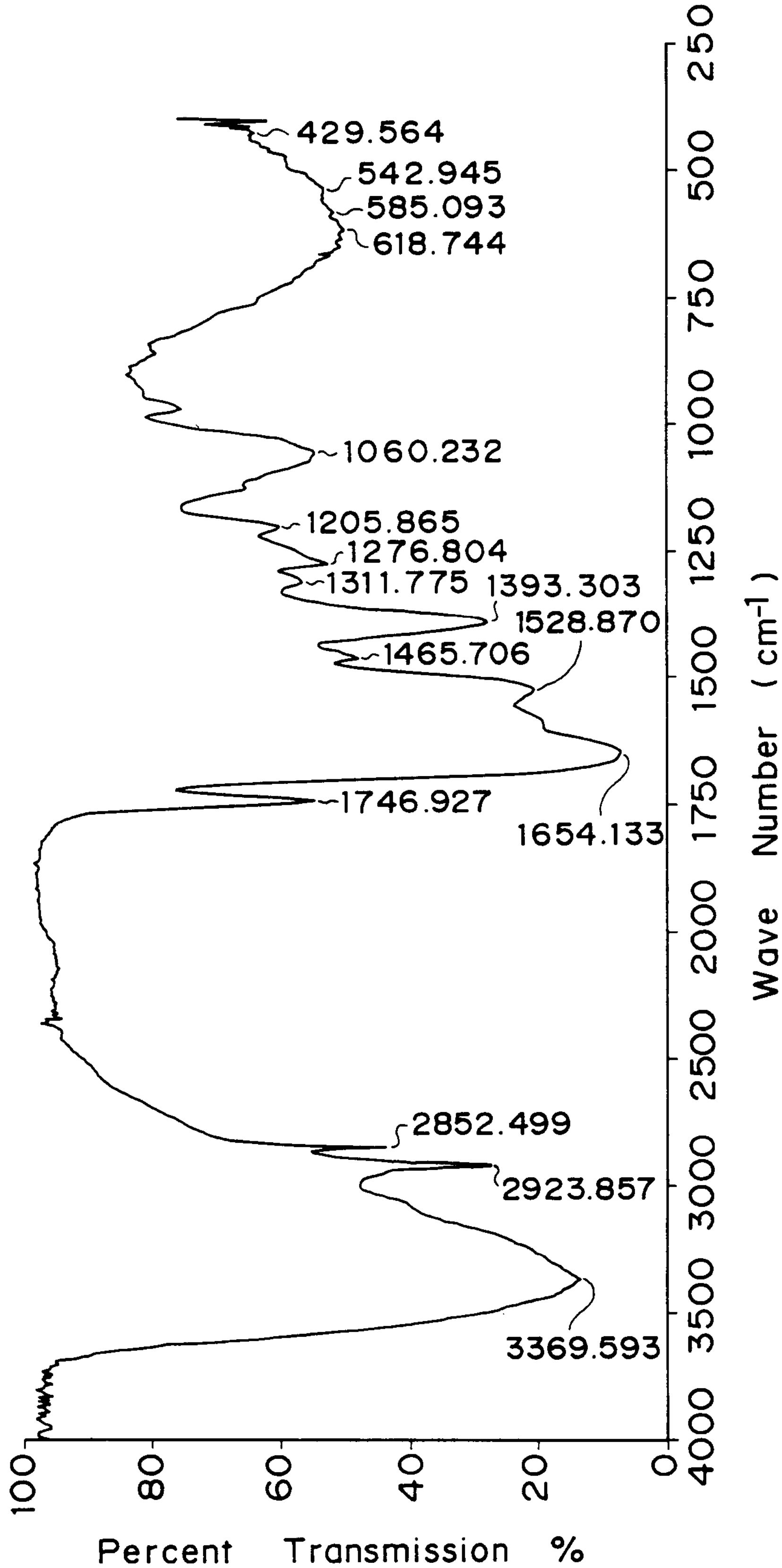
429.564
542.945
585.093
618.744
1060.232
1205.865
1276.804
1311.775
1393.303
1528.870
1465.706
1746.927
1654.133
2852.499
2923.857
3369.593
100
80
60
40
20
0
Percent Transmission %
4000
3500
3000
2500
2000
1750
1500
1250
1000
750
500
250
Wave Number ( cm$^{-1}$ )

Fig. 8

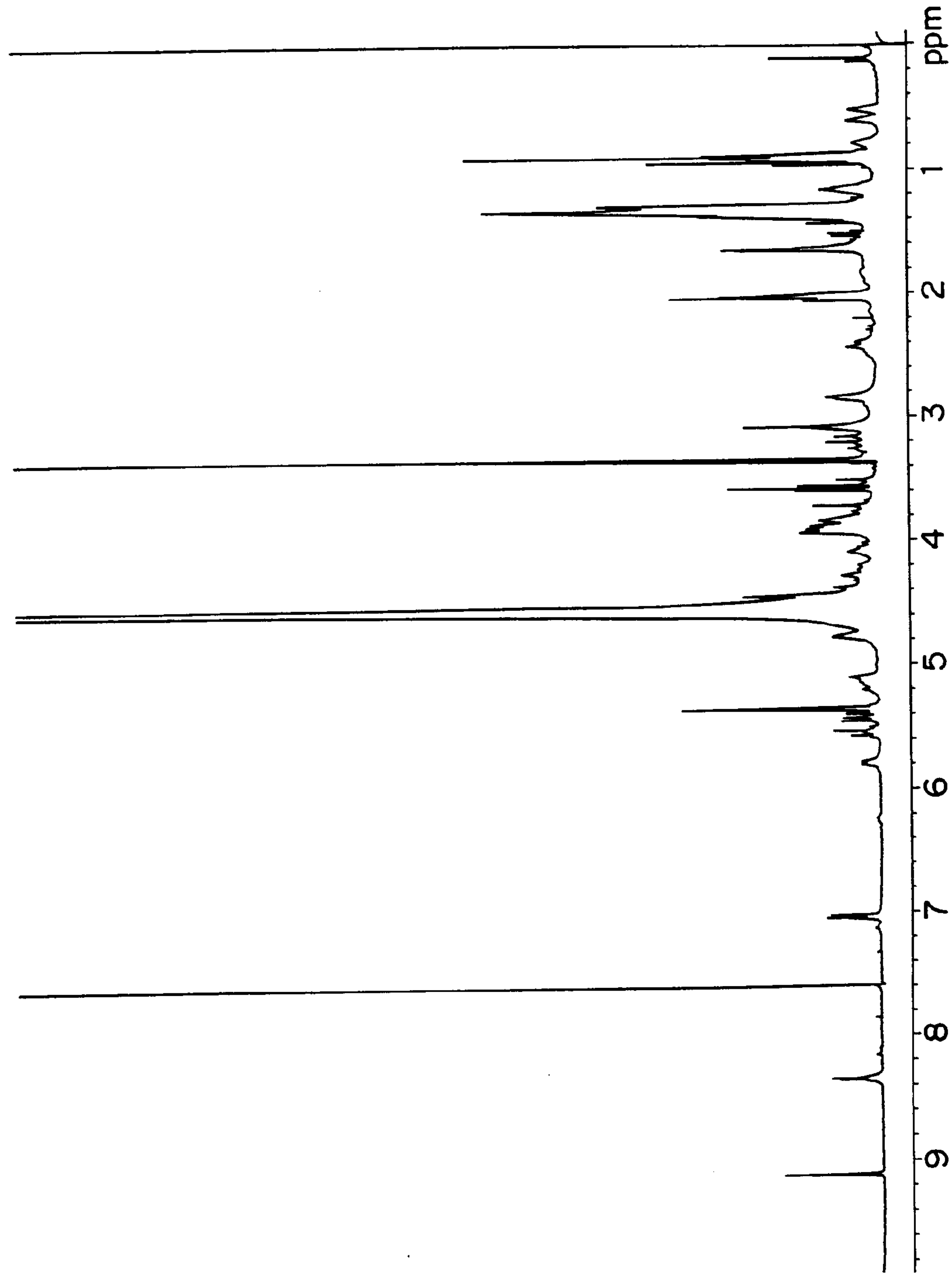

Fig. 9

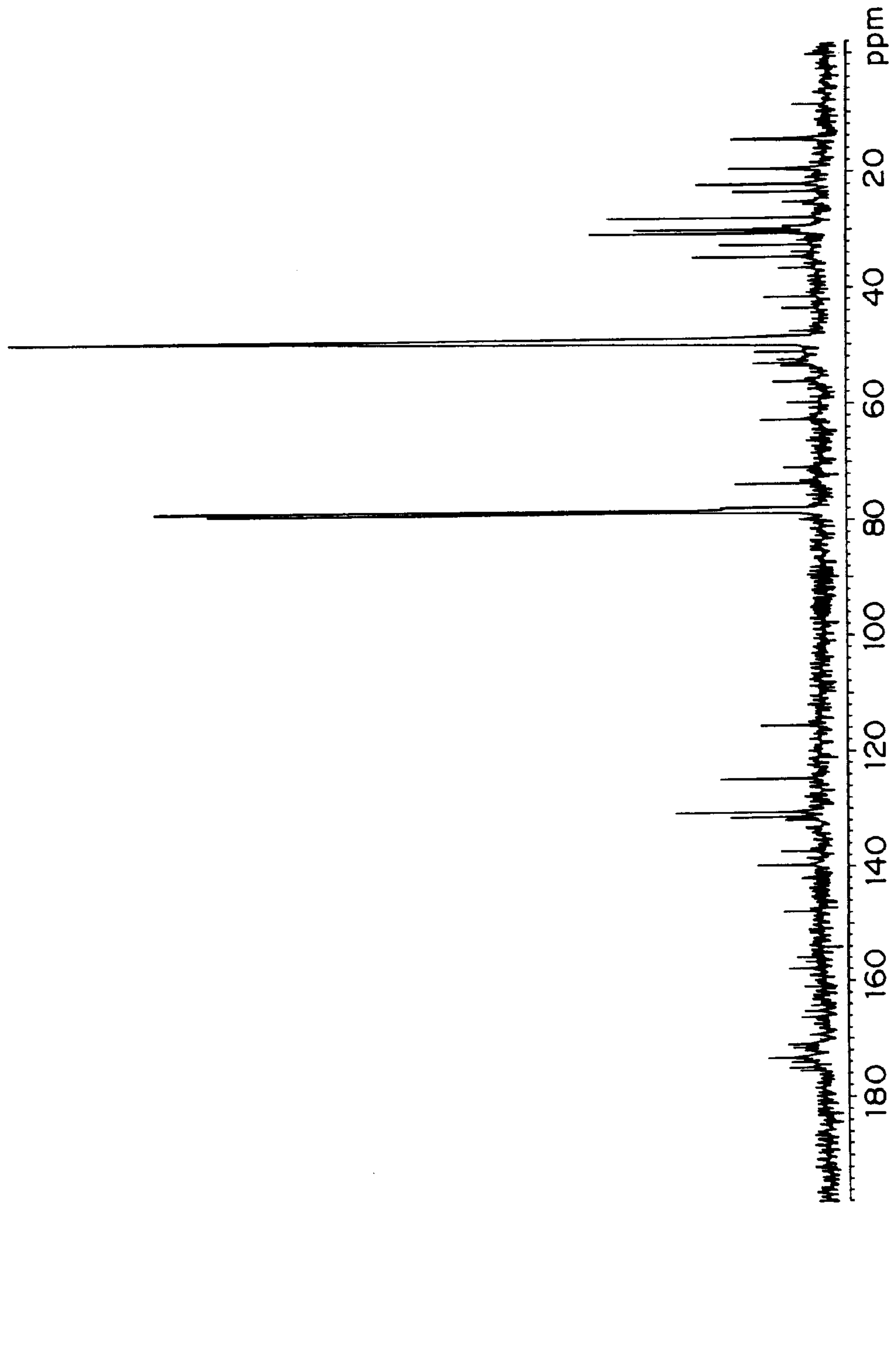

Fig.10

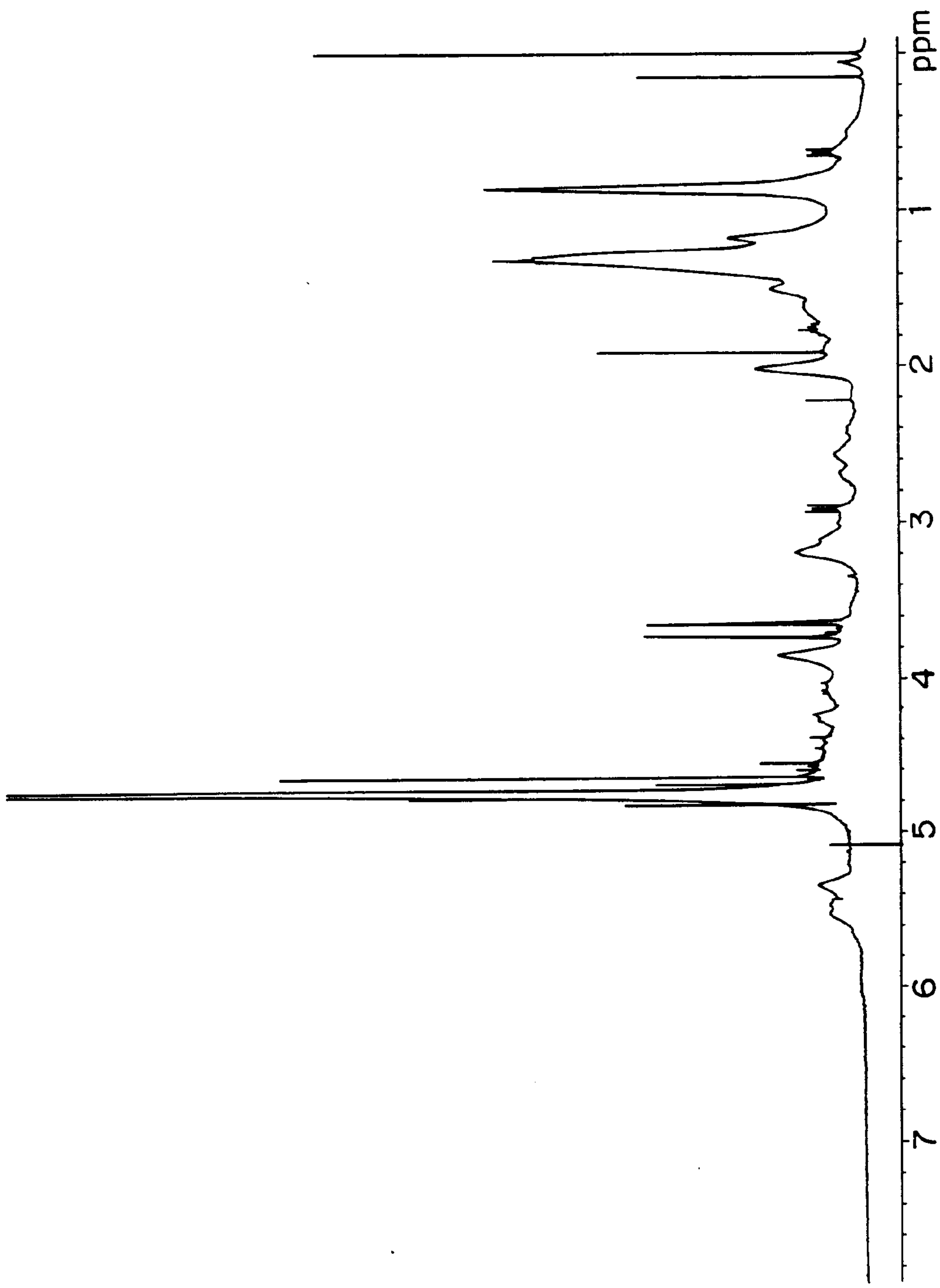

Fig.11

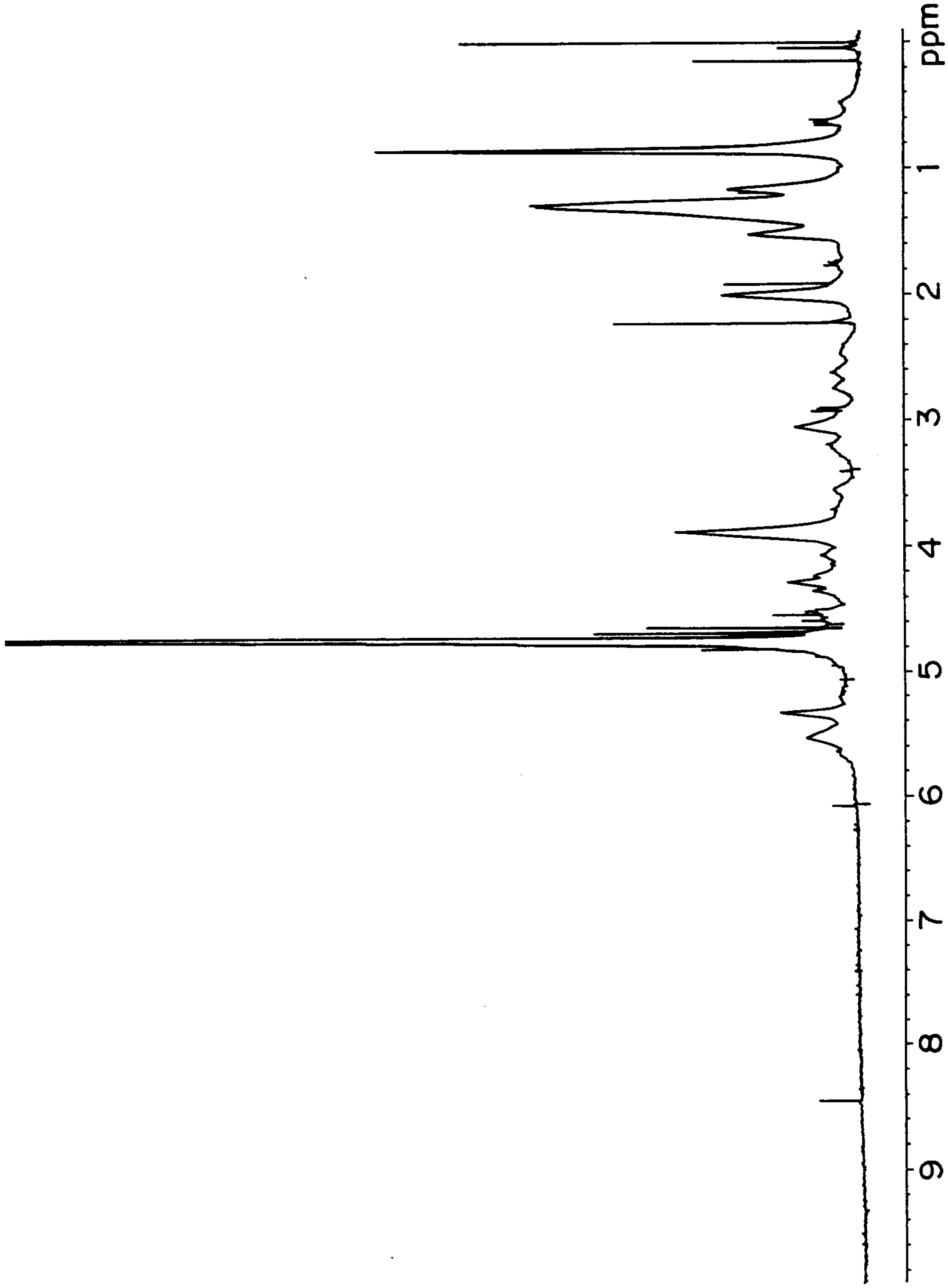

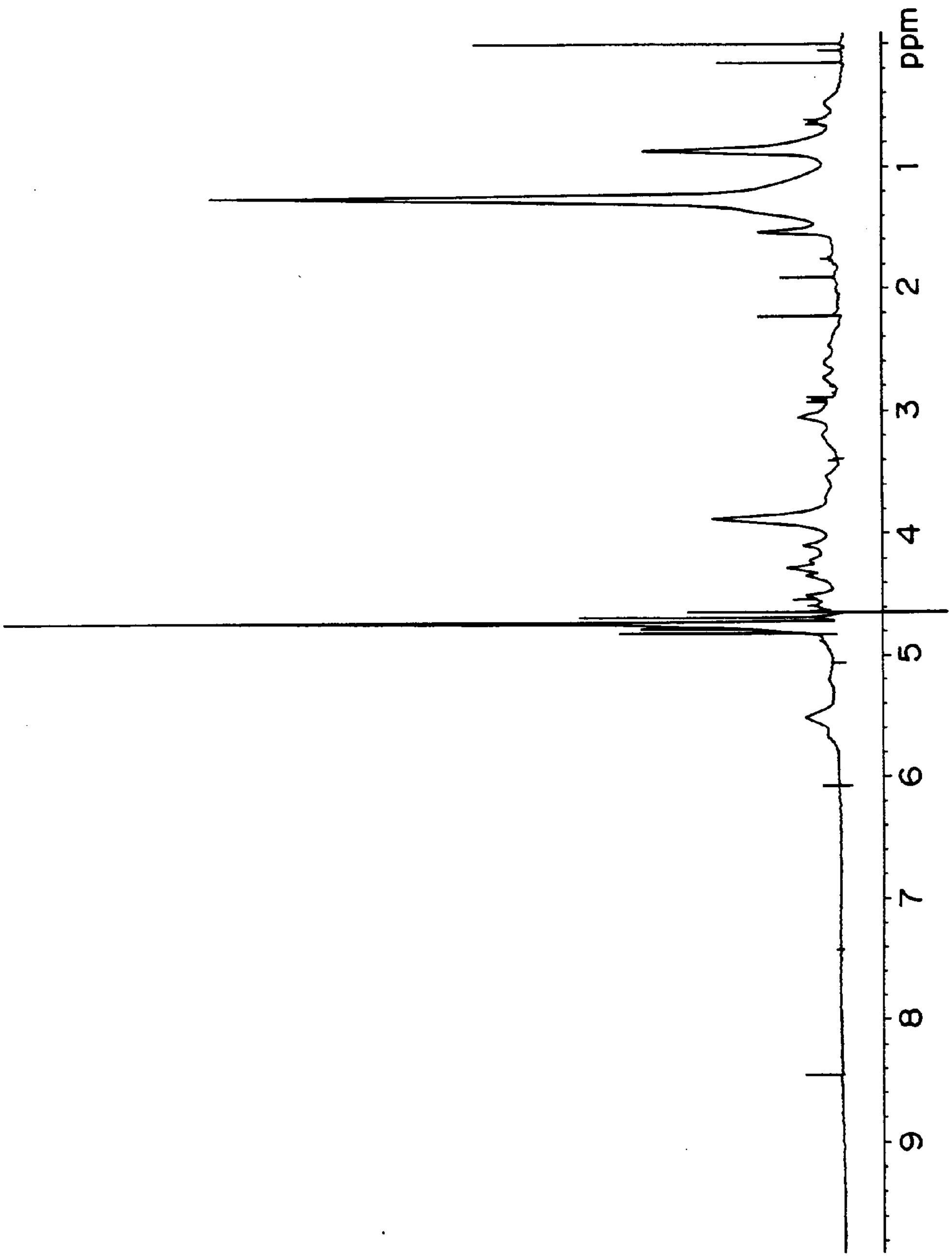


Fig.12

Fig. 13

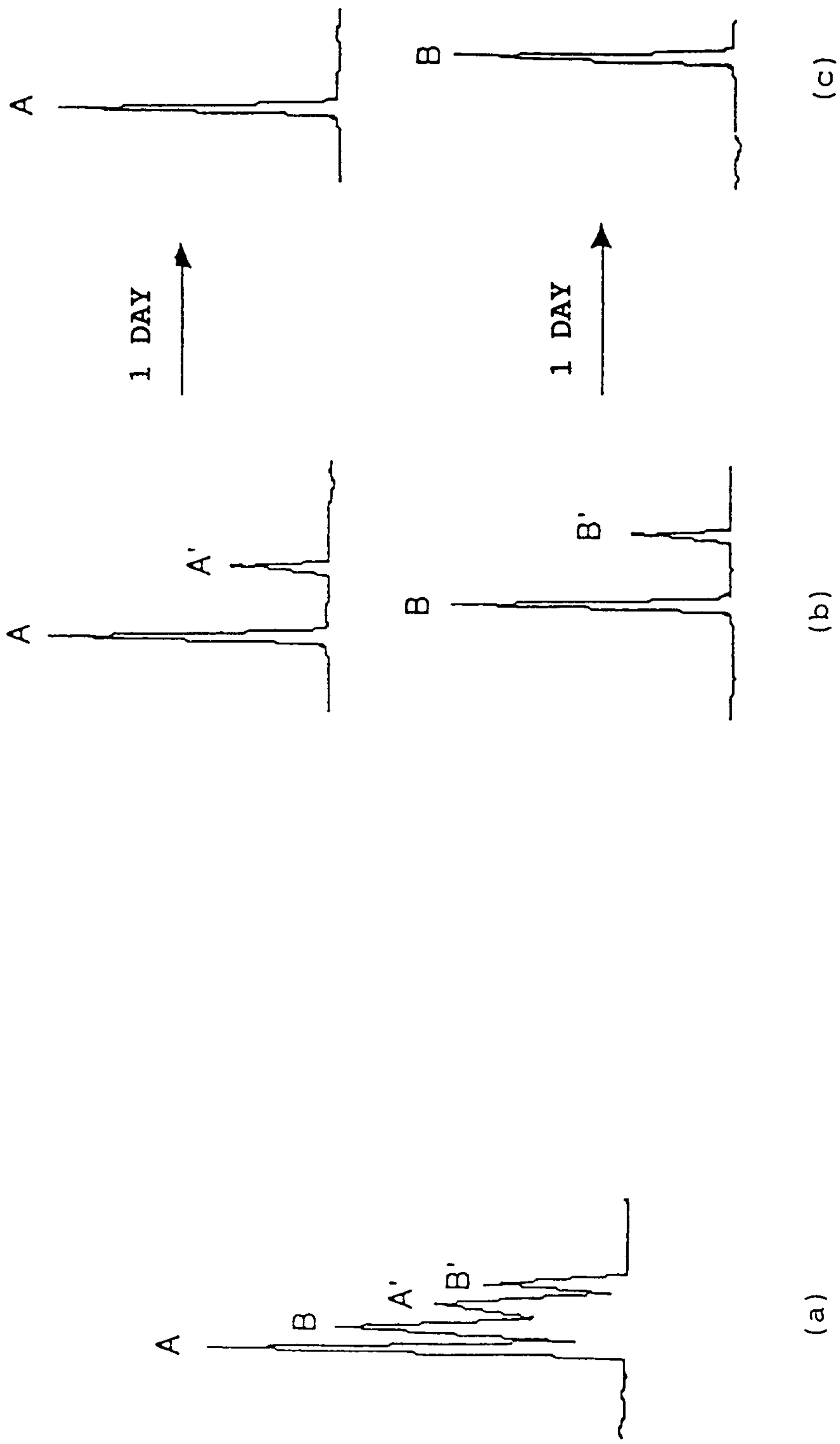

European Patent Office

# EUROPEAN SEARCH REPORT

Application Number EP 93306513.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | PATENT ABSTRACTS OF JAPAN, unexamined applications, C field, vol. 15, no. 80, February 25, 1991 THE PATENT OFFICE JAPANESE GOVERNMENT page 119 C 810 * No. 2-303 496 (SHIONOGI & CO) * -- | 1-5 | C 12 P 21/00<br>A 61 K 37/02<br>//(C 12 P 21/00<br>C 12 R 1:38) |
| A | EP - A - 0 413 967 (SHIONOGI SEIYAKU KABUSHIKI KAISHA) * Totality * ---- | 1-5 | |

TECHNICAL FIELDS SEARCHED (Int. Cl.5)

C 12 P
A 61 K

The present search report has been drawn up for all claims

| Place of search | Date of completion of the search | Examiner |
|---|---|---|
| VIENNA | 09-11-1993 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)